# EUROPEAN PATENT SPECIFICATION

(11) **EP 0 363 913 B1**
(45) Date of publication and mention of the grant of the patent: **26.01.1994**
(21) Application number: 89118828.6
(22) Date of filing: 10.10.1989
(51) Int. Cl.: A61M 15/00, A62B 21/00

(54) **Reusable oxygen inhaler**
Wiederverwendbarer Sauerstoffinhalator
Inhalateur d'oxygène, réutilisable

(30) Priority: 11.10.1988 JP 253801/88
(43) Date of publication of application: 18.04.1990
(73) Proprietor: MIDORI ANZEN KOGYO CO. LTD., Shibuya-ku Tokyo (JP)
(72) Inventor: Saitoh, Toshiaki, Kasukabe-shi Saitama-ken (JP); Katoh, Yoshimasa, Machida-shi Tokyo (JP)
(74) Representative: Grünecker, Kinkeldey, Stockmair & Schwanhäusser Anwaltssozietät

(56) References cited:
- US-A- 3 955 931
- US-A- 4 671 270

## Description

### BACKGROUND OF THE INVENTION

### Field of the Invention

The present invention relates to an oxygen inhaler (resuscitator) utilizing an oxygen generating means usually called a chlorate candle which contains an oxygen generating agent primarily composed of sodium chlorate.

### Description of the Background Art

Conventionally, a portable oxygen inhaler utilizing a chlorate candle comprises a chlorate candle arranged inside a metallic outer casing with an thermal insulation layer in between. In such an oxygen inhaler, inside of the outer casing is further equipped an ignition device for starting the oxygen generating reaction of oxygen generating agent contained in the chlorate candle, and an absorbent for absorbing secondary gases generated by the oxygen generating agent.

Such a conventional oxygen inhaler is designed to be of one time use only, so that after the oxygen generation has been completed, the entire device including ignition device is to be discarded altogether, which is quite uneconomical.

In order to cope with this situation, it has been proposed to make the chlorate candle to be freely separable from the outer casing, so that only the chlorate candle needs to be replaced when the device is used once.

However, since the chlorate candle heats up to several hundred °C immediately after the completion of the oxygen generation, the replacement of the chlorate candle by hands are difficult as well as dangerous.

From US-A-4 671 270, there is known an oxygen inhaler, comprising an outer casing and an oxygen generating core to be inserted into the outer casing including oxygen and generating means containing an oxygen generating agent for undergoing oxygen generating reaction, an ignition portion for starting the oxygen generating reaction of the oxygen generating agent, and an oxygen outlet for outpouring the generated oxygen. Furthermore, the oxygen generating core includes a gypsum layer surrounding at least a portion of a side face of the oxygen generating means.

The oxygen inhaler of US-A-4 671 270 has the disadvantage that replacing the chlorate candle is complicated or even impossible.

It is therefore, an object underlying the invention to provide an oxygen inhaler in which an accurate and quick attaching of the chlorate candle to the outer casing when replacing the candle, is achieved.

This object is achieved by the characterising features of claim 1.

According to the present invention, there is provided an oxygen inhaler in which a chlorate candle is replaceable so that the oxygen inhaler can be used repeatedly by simply replacing the chlorate candle, and in which the replacement of the chlorate candle can be done easily and accurately without danger.

The subclaims contain preferred embodiments of the present application.

Other features and advantages of the present invention will become apparent from the following description taken in conjunction with the accompanying drawings.

### BRIEF DESCRIPTION OF THE DRAWINGS

Fig. 1 is a vertical cross sectional view of an oxygen generating core of one embodiment of an oxygen inhaler according to the present invention.

Fig. 2 is another vertical cross sectional view of an oxygen generating core of one embodiment of an oxygen inhaler according to the present invention, for explaining an extra feature to be added to the oxygen generating core of Fig. 1.

Fig. 3 is a transverse cross sectional view of the oxygen generating core of Fig. 2.

Fig. 4 is a vertical cross sectional view of one embodiment of an oxygen inhaler according to the present invention, using the oxygen generating core of Figs. 2 and 3.

### DETAILED DESCRIPTION OF THE PREFERRED EMBODIMENTS

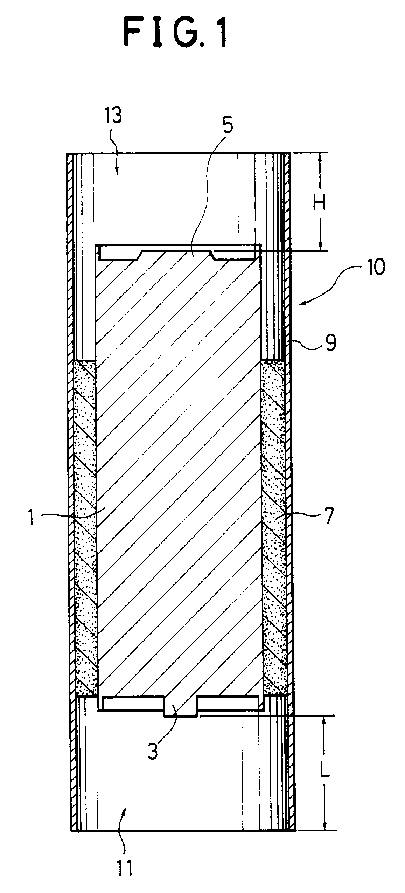

Referring now to Figs. 1 to 4, there is shown one embodiment of an oxygen inhaler according to the present invention.

Fig. 1 shows a cylindrical oxygen generating core 10 incorporating a cylindrical chlorate candle 1 which contains an oxygen generating agent (not shown) mainly composed of sodium chlorate as well as an absorbent (not shown) for absorbing secondary gases generated by this oxygen generating agent. The chlorate candle 1 further includes an ignition portion 3 for starting the oxygen generating reaction of oxygen generating agent on one end, and an oxygen outlet 5 from which the generated oxygen is to be extracted on the other end. Since this type of chlorate candle with an ignition portion and oxygen outlet is already known in the art, the description of further details of the chlorate candle 1 with the ignition portion 3 and the oxygen outlet 5 will be omitted.

The oxygen generating core 10 further includes a gypsum layer 7 surrounding the chlorate candle 1 around substantial part of an outer side face of the chlorate candle 1, and a protection cylinder 9 made of paper material which is provided around the gypsum layer 7 to constitute an outer side face of the oxygen generating core 10. As shown in Fig. 1, the protection cylinder 9 has a height greater than that of the chlorate candle 1. As a consequence, there are spaces 11 and 13 above and below the chlorate candle 1, respectively, formed inside the protection cylinder 9. Furthermore, in this oxygen generating core 10, the chlorate candle 1 is located with respect to the protection cylinder such that a distance H between upper ends of the protection cylinder 9 and the chlorate candle 1 substantially differs from a distance L between lower ends of the protection cylinder 9 and the chlorate candle 1.

This oxygen generating core 10 is to be inserted into an outer casing in a manner to be explained below, and will be replaced by a new one when the oxygen generation is finished, so that the oxygen inhaler can be made reusable by simply replacing the oxygen generating core 10. Here, the spaces 11 and 13 of each new oxygen generating core 10 may be used to install a new oxygen mask to be used in conjunction with the oxygen inhaler, so that the oxygen mask will also be replaced every time the oxygen generating core 10 is replaced, which is desirable from the hygienic point of view.

Now, during the oxygen generation by the oxygen generating core 10, the chlorate candle 1 is heated up to several hundred °C by the heat generated as the oxygen generating agent inside the chlorate candle undergoes the oxygen generating reaction. Meanwhile, as the temperature of the chlorate candle 1 increases, the gypsum layer 7 surrounding the chlorate layer 1 undergoes transformation from gypsum to plaster of Paris at 128 °C, and then to calcium sulfate anhydride at 163 °C, in a course of which the crystallized water will be separated and subsequently evaporated from the gypsum layer 7. Such water separated from the gypsum layer 7 will then be absorbed by the protection cylinder 9 which is water adsorptive as it is made of paper material, and then evaporated from this protection cylinder 9 toward outside of the oxygen generating core 10. Consequently, the surface of the protection cylinder 9 will be cooled down to below one hundred °C by means of the heat released by the evaporation of water. In particular, as the ends of the protection layer 9 are separated by substantial distances from the chlorate candle 1, these ends of the protection cylinder 9 can remain at relatively low temperature.

Thus, in this oxygen generating core 10, a very hot chlorate candle 1 can be handled without danger by handling the relatively cool ends of the protection cylinder 9, without necessitating a dangerous direct contact with the chlorate candle 1.

Now, although the evaporation of water from the gypsum layer 7 through the protection cylinder 9 is effective to reduce the temperature of the outer side face of the oxygen generating core 10 to below one hundred °C, this temperature may still be too high for a direct contact without danger.

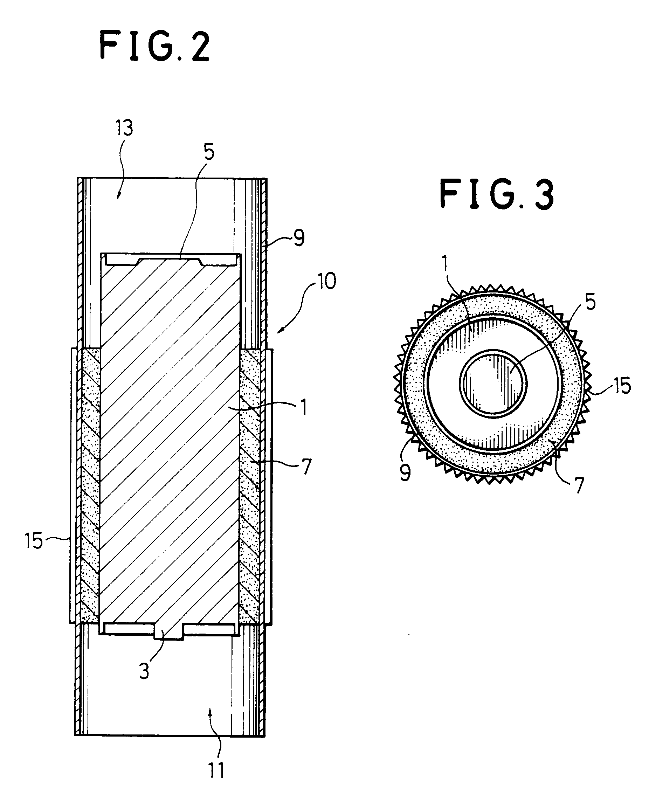

For this reason, the oxygen generating core 10 is further equipped, as shown in Figs. 2 and 3, with an insulating member 15 covering a region of the protection cylinder 9 where the gypsum layer 7 and the protection cylinder 9 are in direct contact. The insulating member 15 is made of a corrugated cardboard rolled in cylindrical form, as shown in Figs. 2 and 3, so that there is an air layer between the protection cylinder 9 and the insulating member 15, and also that a surface area of the insulating member 15 can be made large, both of which contribute to a better insulating effect. Moreover, because of such a corrugated shape of the insulating member 15, a direct contact to the oxygen generating core 10 at the insulating member 15 is possible only line-wise, so that the area of contact can be kept small.

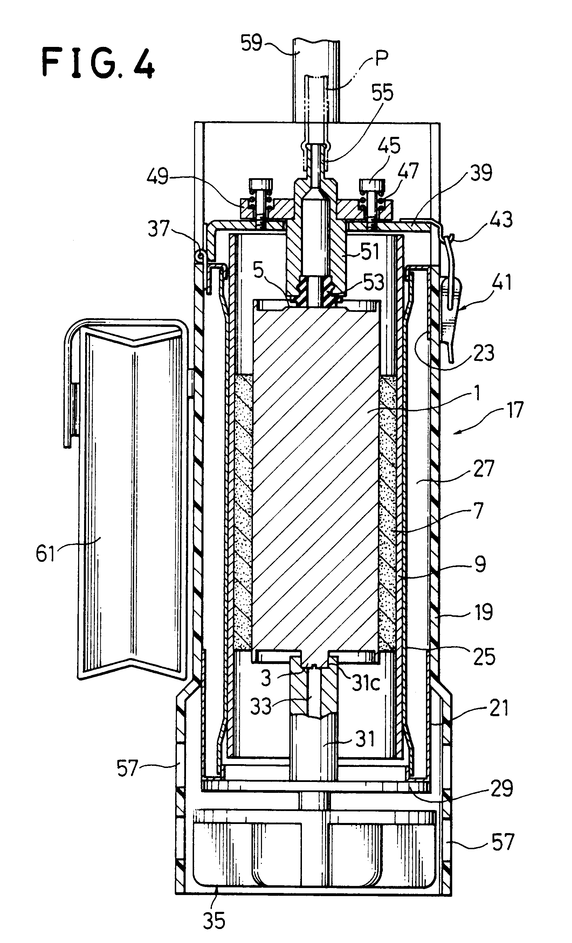

Referring now to Fig. 4, the outer casing of this embodiment of the oxygen inhaler will be described.

In Fig. 4, the oxygen inhaler 17 comprises the cylindrical outer casing 19 in which the oxygen generating core 10 is installed. The oxygen generating core 10 is supported inside the outer casing 19 by means of a lower and an upper brackets 21 and 23, respectively, both of which are attached to an inner side face of the outer casing 19 and a cylindrical supporting member 25 held by the lower and upper brackets 21 and 23, such that there is a spacing 27 formed between the outer casing 19 and the supporting member 25.

At the bottom of the outer casing 19, there is a frame 29 attached to the lower bracket 21, and at a middle of the frame 29 there is supported a pin holder 31 having a concaved top 31c to be engaged with the ignition portion 3 of the chlorate candle 1. In the pin holder 31, there is provided a striker pin 33 for striking the ignition portion 3 to ignite it, so that the ignition portion 3 can start the oxygen generating reaction of the oxygen generating agent inside the chlorate candle 1. The striker pin 33 is controlled by operating a striker device 35 provide underneath the frame 29. This striker device 35 can be of any known device for making the striker pin 33 to hit the ignition portion 3 with some impulse, such as that which utilizes an abrupt release of deformation impressed on an elastic body.

On the other hand, at the top of the outer casing 19, there is an openable lid 39 attached to the outer casing 19 by a hinge 37, which can freely be locked and unlocked by means of a locking device 41 with a hook 43 provided on the outer side face of the outer casing 19. On top of this lid 39, there is a movable plate 49 pressed to the lid 39 with a number of bolts 45 with springs 47 in between, and a middle of this movable plate 49 is pierced through by a manifold 51. The manifold 51 is euqipped with a nipple 53 at a bottom which can freely be attached to the oxygen outlet 5 of the chlorate candle 1, and a pipe connector 55 at a top to be connected with a pipe P which connects the oxygen inhaler 17 and an external oxygen mask (not shown) from which the oxygen is to be inhaled.

The oxygen inhaler 17 further incorporates a number of ventilation holes 57 on its side wall for allowing air to fill the spacing 27, a handle piece 59 on its top for facilitating an easy carriage of the oxygen inhaler 17, and a container 61 for storing oxygen masks and other equipments associated with the oxygen inhaler 17.

The oxygen can be generated by this oxygen inhaler 17 by operating the striker device 35 to make the striker pin 33 to ignite the ignition portion 3 of the chlorate candle 1. During the oxygen generation by the oxygen generating core 10, because of the aforementioned merit of the oxygen generating core 10 to maintain the temperature of the protection cylinder 9 relatively low, and also because of the cooling of the outer casing 19 by the air filling the spacing 27, the outer casing 19 can be kept at a safely low temperature.

The oxygen generated by the oxygen generating core 10 will pour from the oxygen outlet 5 of the chlorate candle 1 through the nipple 53 out to the manifold 51, from which the oxygen flows through the pipe connector 55 and the pipe P to the external oxygen mask to be given to an asphyxiated patient.

When the oxygen generation is finished, the lid 39 is opened by releasing the lock device 41, so that the oxygen generating core 10 can be lifted out of the outer casing 19 by pulling the upper end of the protection cylinder 9 upwards. A new oxygen generating core 10 will then be inserted into the outer casing 19 as a replacement. Here, because of the different distances H and L between the top ends of the protection cylinder 9 and the chlorate candle 1 and between the bottom ends of the protection cylinder 9 and the chlorate candle 1, respectively, if the new oxygen generating core 10 is inserted upside down, this chlorate candle 1 will stop before fully inserted into the outer casing 19, as the oxygen outlet 5 of the chlorate candle 1 hits the pin holder 31. In such a case the bottom end of the protection cylinder 9 will stick out upwards so much that closing of the lid 39 is obstructed, such that the fact that the oxygen generating core 10 has been inserted upside down can easily be recognized.

As explained, according to the present invention it is possible to provide an oxygen inhaler in which a chlorate candle is replaceable, so that the oxygen inhaler can be used repeatedly by simply replacing the chlorate candle, and in which the replacement of the chlorate candle can be done easily and accurately without danger.

It is to be noted that although the lid 39 is openable with respect to the outer casing while the striker device 35 is fixed in the above embodiment, this feature can be replaced by an openable striker device 35 and a fixed lid 39, without affecting the significance of the present invention.

Besides this, many modifications and variations of the above embodiment may be made without departing from the novel and advantageous features of the present invention. Accordingly, all such modifications and variations are intended to be included within the scope of the appended claims.

## Claims

1. An oxygen inhaler, comprising:
an outer casing (19); and
an oxygen generating core (10), to be inserted into the outer casing (19), including:
oxygen generating means (1) containing:
an oxygen generating agent for undergoing oxygen generating reaction;
an ignition portion (3) for starting the oxygen generating reaction of the oxygen generating agent; and
an oxygen outlet (5) for outpouring the generated oxygen;
said oxygen generating core (10) further including:
a gypsum layer (7) surrounding at least a portion of a side face of the oxygen generating means (1);
**characterised in that**
said oxygen generating core (10) comprises water absorptive protection cylinder means (9) for surrounding the outer side face of the gypsum layer (7), having such a height that the height difference between the top ends of the oxygen generating means (1) and the protection cylinder means (9) is substantially different from the height difference between the bottom ends of the oxygen generating means (1) and the protection cylinder means (9);
and also characterised in that the outer casing (19) further includes:
a supporting member (25) for supporting the oxygen generating core (10) inside the outer casing (19) with a spacing (27) formed between the supporting member (25) and the inner side face of the outer casing (19), such that the oxygen generating core (10) is supported inside the outer casing (19) to be replaceable with a new oxygen generating core after the generation of oxygen is finished.

2. The oxygen inhaler of claim 1, further comprising a heat insulating member (15) surrounding at least a portion of a side face of the protection cylinder means (9).

3. The oxygen inhaler of claim 2, wherein the heat insulating member (15) has a corrugated shape.

4. The oxygen inhaler of one of the claims 1-3, wherein the outer casing (19) further includes:
ventilation holes (57) for connecting the spacing (27) formed between the supporting member (25) and the inner side face of the outer casing (19) with outside of the oxygen inhaler.

5. The oxygen inhaler of claim 1, wherein the outer casing further includes:
a striker means (33), provided on one end of the outer casing (19), for striking the ignition portion (3) of the oxygen generating core (10) to ignite it; and
manifold means (51), provided on another end of the outer casing (19), to be connected with the oxygen outlet (5), for leading the generated oxygen from the oxygen outlet (5) to outside of the oxygen inhaler;
and wherein one of the striker means (33) and the manifold (51) is openable with respect to the outer casing (19).

## Patentansprüche

1. Sauerstoffinhalator, mit:
einem äußeren Gehäuse (19); und
einem Sauerstofferzeugungskern (10), zum Einfuhren in das äußere Gehäuse (19), der umfaßt:
eine Sauerstofferzeugungseinrichtung (1), die enthält:
einen Sauerstofferzeugungsstoff, der sich einer Sauerstofferzeugungsreaktion unterzieht;
einen Zündbereich (3) zum Starten der Sauerstofferzeugungsreaktion des Sauerstofferzeugungsstoffes; und
einen Sauerstoffauslaß (5) zum Ausgeben des erzeugten Sauerstoffs;
wobei der Sauerstofferzeugungskern (10) weiterhin umfaßt:
eine Gipsschicht (7), die wenigstens einen Bereich einer Seitenfläche der Sauerstofferzeugungseinrichtung (1) umgibt;
**dadurch gekennzeichnet,**
daß der Sauerstofferzeugungskern (10) eine Wasserabsorptionsschutzzylindereinrichtung (9) aufweist, die die Außenseite der Gipsschicht (10) umgibt, wobei sie eine derartige Höhe aufweist, das die Höhendifferenz zwischen den oberen Enden der Sauerstofferzeugungseinrichtung (1) und der Schutzzylindereinrichtung (9) sich wesentlich von der Höhendifferenz zwischen den Bodenenden der Sauerstofferzeugungseinrichtung (1) und der Schutzzylindereinrichtung (9) unterscheiden;
und ebenso dadurch gekennzeichnet, daß das äußere Gehäuse (19) weiterhin aufweist:
ein Stützteil (25) zum Stützen des Sauerstofferzeugungskerns (10) innen im äußeren Gehäuse (19) mit einem Spalt (27), der zwischen dem Stützteil (25) und der Innenseite des äußeren Gehäuses (19) ausgebildet ist, derart, daß der Sauerstofferzeugungskern (10) in dem Gehäuse (19) so gestützt wird, daß er mit einem neuen Sauerstofferzeugungskern ausgetauscht werden kann, nachdem die Erzeugung des Sauerstoffs beendet ist.

2. Sauerstoffinhalator nach Anspruch 1, der weiterhin ein Wärmeisolierteil (15) aufweist, das wenigstens einen Bereich der Seitenfläche der Schutzzylindereinrichtung (9) umgibt.

3. Sauerstoffinhalator nach Anspruch 2, bei dem das Wärmeisolierteil (15) eine gewellte oder gerippte Form aufweist.

4. Sauerstoffinhalator nach einem der Ansprüche 1 bis 3, bei dem das äußere Gehäuse (19) weiterhin aufweist:
Lüftungsöffnungen (57) zum Verbinden des Spaltes (27), der zwischen dem Stützteil (25) und der inneren Seitenoberfläche des äußeren Gehäuses (19) gebildet ist, mit der Außenseite des Sauerstoffinhalators.

5. Sauerstoffinhalator nach Anspruch 1, wobei das äußere Gehäuse weiterhin aufweist:
eine Aufpralleinrichtung (33), die an einem Ende des äußeren Gehäuses (19) vorgesehen ist, zum Aufprallen auf den Zündbereich (3) des Sauerstofferzeugungskerns (10), um diesen zu zünden; und
einer Membraneinrichtung (51), die an dem anderen Ende des äußeren Gehäuses (19) vorgesehen ist, um mit dem Sauerstoffauslaß (5) verbunden zu werden, um den erzeugten Sauerstoff von dem Sauerstoffauslaß (5) zu der Außenseite des Sauerstoffinhalators zu leiten; und, wobei entweder die Aufpralleinrichtung (33) oder die Membran (51) bezüglich des äußeren Gehäuses (19) geöffnet werden kann.

## Revendications

1. Inhalateur d'oxygène, comprenant
une enveloppe extérieure (19) ;et
une partie centrale (10) génératrice d'oxygène, destinée à être insérée dans l'enveloppe extérieure (19), comprenant :
un moyen générateur d'oxygène (1) contenant :
un agent générateur d'oxygène destine à subir une réaction de production d'oxygène ;
une partie d'amorçage (3) pour amorcer la réaction de production d'oxygène de l'agent générateur d'oxygène ;
et
une sortie d'oxygène (5) pour l'écoulement de l'oxygène produit ;
ladite partie centrale (10) génératrice d'oxygène conprenant en outre ;
une couche de gypse (7) entourant au moins une partie de la face latérale du moyen générateur d'oxygène (1) ;
caractérisé en ce que
ladite partie centrale (10) génératrice d'oxygène comprend un moyen formant cylindre de protection (9), absorbeur d'eau, entourant la face latérale extérieure de la couche de gypse (7), et ayant une hauteur telle que la différence de hauteur entre les extrémités supérieures du moyen générateur d'oxygène (1) et du moyen formant cylindre de protection (9) est sensiblement différente de la différence de hauteur entre les extrémités inférieures du moyen générateur d'oxygène (1) et du moyen formant cylindre de protection (9) ;
et également caractérisé en ce que l'enveloppe extérieure (19) comprend en outre :
un élément de support (25) pour supporter la partie centrale (10), génératrice d'oxygène, à l'intérieur de l'enveloppe extérieure (19) avec un espace (27) formé entre l'élément de support (25) et la face latérale intérieure de l'enveloppe extérieure (19), de sorte que la partie centrale (10) génératrice d'oxygène est supportée à l'intérieur de l'enveloppe extérieure (19) de manière à pouvoir être remplacée par une nouvelle partie centrale génératrice d'oxygène, une fois la production d'oxygène est terminée.

2. Inhalateur d'oxygène selon la revendication 1, comprenant en outre un élément calorifuge (15) entourant au moins une partie de la face latérale du moyen formant cylindre de protection (9).

3. Inhalateur d'oxygène selon la revendication 2, dans lequel l'élément calorifuge (15) a une forme ondulée.

4. Inhalateur d'oxygène selon l'une des revendications 1 à 3, dans lequel l'enveloppe extérieure (19) comprend en outre :
des trous de ventilation (57) pour relier l'espace (27) formé entre l'élément de support (25) et la face latérale intérieure de l'enveloppe extérieure (19) avec l'extérieur de l'inhalateur d'oxygène.

5. Inhalateur d'oxygène selon la revendication 1, dans lequel l'enveloppe extérieure comprend en outre :
un moyen percuteur (33), placé sur une extrémité de l'enveloppe extérieure (19), destiné à percuter la partie d'amorçage (3) de la partie centrale (10) génératrice d'oxygène pour l'amorcer ; et
une tubulure (51), placée sur une autre extrémité de l'enveloppe extérieure (19), devant être raccordée à la sortie d'oxygène (5), pour conduire l'oxygène produit depuis la sortie d'oxygène (5) vers l'extérieur de l'inhalateur d'oxygène ;
et dans lequel le moyen percuteur (33) et la tubulure (51) peuvent l'un ou l'autre s'ouvrir par rapport à l'enveloppe extérieure (19).
